Europäisches Patentamt

European Patent Office

Office européen des brevets

(19)

(11) **EP 1 249 440 A1**

(12) **EUROPEAN PATENT APPLICATION**

(43) Date of publication:
**16.10.2002 Bulletin 2002/42**

(51) Int Cl.⁷: **C07C 15/107**, C07C 2/66,
B01J 29/18

(21) Application number: **01108956.2**

(22) Date of filing: **10.04.2001**

(84) Designated Contracting States:
**AT BE CH CY DE DK ES FI FR GB GR IE IT LI LU
MC NL PT SE TR**
Designated Extension States:
**AL LT LV MK RO SI**

(71) Applicant: **Taiwan Styrene Monomer Corporation
Taipei (TW)**

(72) Inventor: **Wang, Ikai
Hsinchu, Taiwan, 300 (TW)**

(74) Representative: **Grünecker, Kinkeldey,
Stockmair & Schwanhäusser Anwaltssozietät
Maximilianstrasse 58
80538 München (DE)**

(54) **Process for preparing linear alkylbenzenes**

(57)     The present invention discloses a modified mordenite catalyst having an improved stability in an alkylating process of benzene with a long chain olefin. The modified mordenite catalyst is prepared either by a mild steam treatment or by a chemical vapor deposition of Si. It is believed that the former will result in a dealumination of the parent mordenite and the latter will cause the acid sites on the surface of the parent mordenite blocked by Si deposited, both of which enhance the stability of the modified catalyst without sacrificing the activity and selectivity of the modified mordenite catalyst.

Figure 2

EP 1 249 440 A1

**Description**

Field of the Invention

The present invention is related to a process for alkylating benzene with a long chain olefin over a modified mordenite catalyst, and in particular to a process for alkylating benzene with a long chain olefin over a mordenite catalyst modified by a mild steam treatment or by a chemical vapor deposition of Si.

Background of the Invention

The increasing use of detergents causes further release and accumulation of sulphonated alkylbenzene and its derivatives in rivers and streams. Rapid biodegradation of the released organic compounds is highly desired to keep the pollutant as low as possible. Among its isomers, linear alkylbenzene (LAB), the intermediate of detergents, particularly 2-phenyl alkane or 2-phenyl alkane sulphonate is the most biodegradable and environmental favorable isomer.

LAB, which is industrially produced with benzene alkylation, is catalyzed by HF or $AlCl_3$ using $\alpha$-olefin, particularly dodecene, or $AlCl_3$ using chloroalkanes. Those homogenous catalysts are the source of environmental pollution, equipment corrosion, and separation disadvantages such as the difficulty in the manufacturing process after treatment.

It is clear that there is a great need in the detergent industry to develop an environmentally friendly alkylation process capable of not only replacing the conventional $AlCl_3$ or HF catalyst but also having a good selectivity of 2-phenylalkane.

Solid acid catalyst, including zeolite, pillared clay, amorphorous silica-alumina, silicotungstic acid [$SiO_4$($W_3O_9)_4$], and super acid are highly promising for such an environmental friendly process. Universal Oil Products (UOP) announced a new process with a solid acid catalyst in 1990 [Vora, B.V., Pujado, P.R., Imai, T. and Fritsch, T.R., *Chem. Ind*., **19,** 187 (1990)], which was commercialized in Canada in 1995. This process is similar to the process disclosed in US patent No. 5,012,021. Sivasanker and Thangaraj indicated that in terms of selectivity in 2-phenylalkane, HF and Re-Y zeolite are the lowest one in the range of only 14~20% [Sivasanker, S., and Thangaraj, A., *J. Catal*., **138,** 386 (1992); Magnoux, P., Mourran, A., Bernard, S., and Guisnet, M., *Stud. Surf. Sci. Catal*., **108,** 101 (1997)], mordenite is the most outstanding one with a selectivity up to 64%. Meanwhile, most other solid catalysts and $AlCl_3$ have a selectivity range from 26~33%. However, the origin of product selectivity is still unclear. In addition, most solid catalyst has a serious deactivation problem [Da, Z., Magnoux, P., and Guisnet, M., *Appl. Catal. A: General*, **182,** 407 (1999)], thus inhibiting their industrial applications.

Summary of the Invention

The present invention provides an improvement to a process for preparing a linear alkylbenzene in the aspects of catalytic activity, stability and selectivity of 2-phenyl substituted isomer of the catalyst used therein. The process comprises flowing a hydrogen gas and a liquid feed comprising benzene and an olefin through a bed of a mordenite catalyst, wherein the improvement comprises alkylating benzene with a C8-C18 long chain alpha olefin in the presence of a modified mordenite catalyst. The modified mordenite catalyst of the present invention can be a dealuminated mordenite catalyst prepared by flowing a steam-containing gaseous mixture through a bed of mordenite catalyst in particulate form at a temperature ranging from 500 K to 900 K, preferably about 813 K, for a period from 0.5 to 10 hours, preferably about 2 hours, wherein the steam-containing gaseous mixture has a steam partial pressure ranging from 30 to 200 mmHg, preferably about 80 mmHg, and the balance inert gas, preferably air, at room temperature.

In said steam pretreatment, preferably said steam-containing gaseous mixture flows through said bed of mordenite catalyst at a flow rate of 30-1000 SCCC/min per gram of catalyst, and more preferably about 30-200 SCCC/min per gram of catalyst.

Alternatively, the modified mordenite catalyst of the present invention can be a blocked mordenite catalyst prepared by being subjected to a chemical vapor deposition of silicon, which comprises flowing a Si precursor-containing liquid and a carrier gas through a bed of mordenite catalyst at a temperature ranging from 353 K to 473 K, preferably from 393 K to 413 K, for a period from 0.5 to 6 hours, preferably from 1.0 to 5 hours, and wherein the Si precursor-containing liquid comprises an organic solvent or an organic solvent mixture and 0.01 to 1.0 parts of Si $(OC_2H_5)_4$ or $Si(OCH_3)_4$ based on the weight of the organic solvent or the organic solvent mixture. Preferably, the Si precursor-containing liquid comprises 0.02 to 0.2 parts of $Si(OC_2H_5)_4$ based on the weight of the organic solvent mixture comprising 80-99 wt% of benzene and 1-20 wt% of methanol.

Preferably, the alkylation is carried out under the following operating conditions: temperature ranging from 373 K to 523 K, a pressure ranging from 0.5MPa to 5Mpa, a feed weight hourly space rate (WHSV) ranging from 2 to 20 hr$^{-1}$, a molar ratio of benzene to said olefin in said liquid feed ranges between 2:1 and 20:1, and a molar ratio of said hydrogen to the liquid feed ranges between 0.1 and 10.

Preferably, said C8-C18 long chain alpha olefin is 1-deodecene.

Preferably, said mordenite catalyst has a Si/Al ratio of 50-200 prior to the modification, and more preferably a Si/Al ratio of about 90.

Preferably, said mordenite catalyst is in the particulate form having a particle size ranging from 4 to 30 mesh number, and more preferably from 8 to 20 mesh number.

In one of the preferred embodiments, a mordenite catalyst used comprises about 30% by weight of $Al_2O_3$ binder.

Brief Description of the Drawings

Figure 1 shows performance of parent mordenite in the alkylation of benzene with 1-deodecene (P: 300 psig.; Temperature: 353-503K; WHSV: 1.94 hr$^{-1}$, $H_2$/H.C.: 0.63 mol/mol).

Figure 2 shows effect of the mild steam pretreatment on the performance of mordenite in the alkylation of benzene with 1-deodecene according to the present invention.

Figure 3 is a plot of relative first order rate constant of alkylation of benzene with 1-deodecene versus partial pressure of pretreatment stream.

Figure 4 shows effect of the chemical vapor deposition (CVD) of Si pretreatment on the performance of mordenite in the alkylation of benzene with 1-deodecene according to the present invention.

Detailed Description of the Preferred Embodiments

The present invention is more particularly described in the following examples that are intended as illustrative only since numerous modifications and variations therein will be apparent to those skilled in the art, wherein the alkylation of benzene with 1-dodecence catalyzed by a commercial mordenite sample and the analysis of the production mixture were carried out according to the general procedures described in Experimental.

EXPERIMENTAL

A commercial mordenite sample, with Si/Al ratio of 90 containing 30% $Al_2O_3$ binder in 1/8" extrudate, was supplied by Sud Chemie Corporation (Germany). The framework structure of the mordenite was confirmed by powder X-ray diffraction (XRD). Benzene (L.C. grade) 1-dodecene (L.C. grade) and 1-decane (L.C. grade) were obtained from Tedia Chemical Company (USA) and were used without further purification. In addition, 1-decane was used as an internal reference for measuring conversion of 1-dodecene.

Reactions were conducted in a continuous flow, fixed-bed reactor (316 stainless, 3/4 inch, o.d.). The sliced or crashed mordenite samples (8-12, 12-20, 20-30 mesh) mixing with quartz sand (ca. 10-20 mesh) were packed into the reactor. Prior to the reaction, mordenite was calcined in air at 813 K for 8 hr with a heating rate of about 120°C/hr and an air flow rate of 30-100 cc/min per gram of catalyst. The reactor was then cooled in a flowing $N_2$ atmosphere down to the desired reaction temperature. Next, the reaction pressure, flow rate of the gas and liquid were regulated appropriately, a sample at specified intervals was taken for analysis. The products were analyzed using a China Chromatography 8700F with a flame ionization detector and an OV101 packed column. The Hewlett Packard 3390A integrator was used in the data processor. Quantitative analysis of linear alkylbenzenes was performed using a mass spectrometer coupled to a gas chromatograph (GC/MS) equipped with a HP capillary column.

The reaction system in the examples applied high benzene to 1-dodecene ratio. Therefore, conversion was accounted for in terms of 1-dodecene and selectivity of all dodecylbenzene isomers was based on its conversion in molar base. The conversion and selectivity are defined as follows:

$$Conversion = 1 - \frac{dodecene\ wt\%\ in\ Reactor\ Effluent}{dodecene\ wt\%\ in\ Feed}$$

$$Selectivity = \frac{dodecylbenzene\ isomer\ wt\%}{dodecene\ wt\%\ Reacted} * \frac{154}{232}$$

Notably 1-decane does not convert in the reaction conditions applied in this study, it was used as internal standard to secure measurement accuracy.

Control Example 1: parent commercial mordenite

Five grams of 1/8 inch extrudate, 12-20 mesh catalyst were prepared and used as above, which equals to L/D of 3. The operational conditions were: pressure: 300 psig; temperature: 353-503 K; weight hourly space velocity (WHSV): 1.94 $hr^{-1}$; and hydrogen to hydrocarbon molar ratio ($H_2$/HC): 0.63. The feed composition was benzene:1-dodecene:1-decane = 10:1:1. The process was continuously run for about 400 hours, wherein the reaction temperature was raised while the conversion of 1-dodecene dropped below 90%.

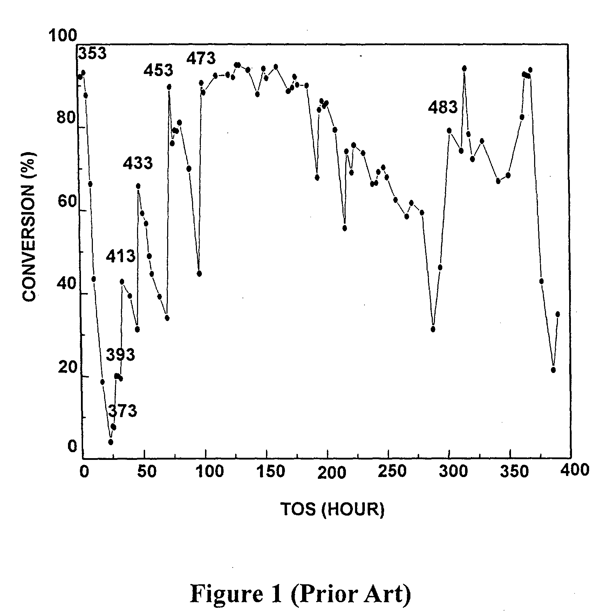

The catalytic performance and reaction trajectory of the parent commercial mordenite in benzene alkylation with dodecene was shown in Fig. 1. Severe deactivation was observed. Reaction temperature was raised to compensate deactivation as time-on-stream increases. The data also show that catalytic activity is more stable at higher reaction temperatures.

Control Example 2 and Example 1: Parent commercial mordenite and steam pretreated mordenite

Five grams of 1/8 inch extrudate, 12-20 mesh catalyst were used in Control Example 2 and Example 1, wherein the catalyst was calcined in air at 813 K for 8 hr in the former case, but in the latter case a gaseous mixture containing air and 30 mmHg steam was introduced to reactor in the last two hours of the 8-hour calcination.

The operational conditions were: pressure: 100 psig; temperature: 398 K; WHSV: 4 $hr^{-1}$; and hydrogen to hydrocarbon molar ratio ($H_2$/HC): 2. The feed composition was benzene: 1-dodecene: 1-decane = 10:1:1.

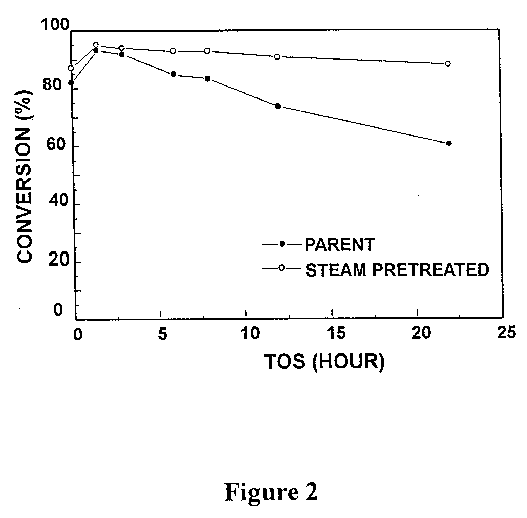

The results are shown in Fig. 2. As it can be seen from Fig. 2, the steam pretreatment procedure greatly improves the catalyst stability.

Example 2: Steam pretreated mordenite using different steam partial pressures

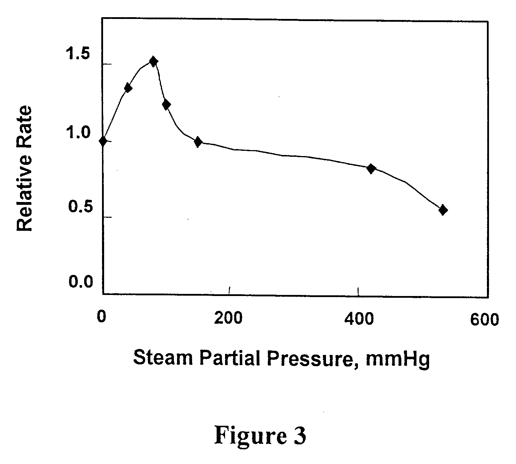

The procedures of Example 1 were repeated except that the catalysts were pretreated with various steam partial pressures. The results are shown in Fig. 3.

According to Fig. 3, the relative first order rate constant initially increases with an increasing partial pressure of steam and reaches a maximum at a partial pressure of 80 mmHg. The low partial pressure treatment can also enhance catalytic stability of mordenite as shown in Fig. 2. More steam pretreatment reduces activity, such as the rate constant of sample pretreated at 530 mmHg is lower than that of parent mordenite.

Example 3: Steam pretreated mordenite and long time-on-stream

The procedures of Example 1 were repeated except that the operational conditions were changed to: pressure: 300 psig; temperature: 433 K; WHSV: 1.92 $hr^{-1}$; and hydrogen to hydrocarbon molar ratio ($H_2$/HC): 0.70. It was found that the steam pretreated catalyst could maintain a high selectivity upto 98% and at a conversion level of 96 % for at least 800 hours.

Among the dodecylbenzene isomers, the 2-isomer and 3-isomer were around 63-67% and 37-33%, respectively as identified with GC/MS spectroscopy. Almost no other isomer was observed. No iso-dodecylbenzene isomer formation occurred as well. The 2-phenyl substituted isomer distribution is substantially higher than catalysts reported elsewhere [Cao, Y., Kessas, R., Naccache, C., and Taarit, Y.B., *Appl. Canal. A: General*, **184,** 231 (1999); Guczi L., "New Frontiers in Catalysis" (Studies in Surface Science and Catalysis, **Vol. 75A),** Elsevier, Amsterdanm, P397 (1992)], it exceeds the thermodynamic equilibrium composition. In comparison, Sivasanker and Thangaraj reported that 2-phenyl isomer distribution in products from most solid catalysts and $AlCl_3$ ranges from 26~33%.

Internal mass transfer resistance was studied from experiments by using different catalyst particle sizes. Catalyst sample of 8-12 mesh particle was found exhibiting a slight faster deactivation than 12-20 mesh particle. According to Table 1, the conversion of 8-12 mesh crushed sample was lower than other smaller crushed samples, implying the existence of internal mass transfer resistance. The conversion of a sliced catalyst sample either in 1/8"x 1/8" or 1/16" x 1/16" was significantly lower than that of a crushed sample (Table 1), it also had a higher deactivation than that of a crushed sample. The discrepancies in performance can be attributed to the high density on the skin of the extrudates. Next, an attempt was made to reduce the mass transfer resistance of skin effect by preparing a high porosity extrudate in 1/16" x 1/16" with an increasing pore opening on the skin. The high porosity sample improved both in terms of 1-dodecence conversion (Table 1) and catalytic stability.

Table 1

| Effect of Intraparticle Diffusion on Dodecene Conversion (Pressure: 450 psig; Temperature: 433K; WHSV: 16 hr$^{-1}$; H$_2$/HC: 0.7 mol/mol; TOS: 20 hr) | | | | | | |
|---|---|---|---|---|---|---|
| | 1/8" Extrudate | | | | 1/16" Extrudate | 1/16" Extrudate (High Porosity) |
| Mesh | 20-30 | 12-20 | 8-12 | Sliced | Sliced | Sliced |
| Conversion | 96% | 96% | 89% | 62% | 52% | 96% |

The inventor also examined the effects of process variables on conversion and selectivity with a series of tests using various reaction temperatures, pressures and feed compositions. Temperature for the process variables studied herein ranged between 353 and 503 K; pressures ranged between 0 and 450 psig; benzene:1-dodecene: 1-decane of feed composition ranged between 5:1:1 to 10:1:1; WHSV ranged between 1.48 and 48 hr$^{-1}$; and H$_2$/feed ratio in between 0.42 and 0.96 mol/mol.

Table 2 summarizes the catalytic results over a temperature range between 373K and 473K under constant pressure, WHSV and feed ratio. The data were collected at a time-on-stream of 6 hr. Conversion increased with reaction temperature. At a reaction temperature of 403K, conversion was 98.5% and dodecylbenzene selectivity reached a maximum of 99%. At a lower reaction temperature of 373K, dodecylbenzene selectivity was 75% with dodecene dimers as the main by-product. At a higher reaction temperature of 473K, dodecylbenzene selectivity dropped to 51% with light alkylbenzenes including propylbenzenes as main by-product.

Table 2

| Dodecene Conversion and Product Selectivity under Various Temperatures over 1/8" Mordenite | | | |
|---|---|---|---|
| Temperature (K) | 373 | 403 | 473 |
| Conversion (%) | 70.3 | 98.5 | 98.6 |
| Product Distribution (wt%) | | | |
| Lights | < 1 | < 1 | 37 |
| Propylbenzenes | < 1 | < 1 | 12 |
| Dodecylbenzene | 75 | 99 | 51 |
| Dodecene Dimers | 22 | < 1 | < 1 |
| Heaviers | 2 | < 1 | < 1 |
| Operating Conditions: Pressure: 300 psig; WHSV: 4 hr$^{-1}$; H$_2$/HC: 0.7 mol/mol; TOS: 6 hr) | | | |

At a low reaction temperature, i.e., 373K, solubility of benzene in 1-dodecene is low, resulting in a significantly higher ratio of 1-dodecene to benzene on catalyst surface than that in reactor feed. Consequently, 1-dodecene proceeds self-polymerization, which is faster than alkylation. At a higher reaction temperature of 473K, due to higher solubility of benzene in 1-dodecene, benzene-to-1-dodecene on catalyst surface increases and oligomerization of 1-dodecene is retarded. As the reaction temperature becomes too high, i.e., 473K, dodecene cracks into smaller fragments, and benzene alkylation with small carbon chains particularly propylene, in which the main by-product is propylbenzene.

It was found that the effect of reaction pressure on conversion and catalyst stability is negligible in the pressure range between 300 psig and 100 psig. The conversion reached up 95% when reaction pressures exceeded 1.0 MPa. Notably, the catalyst deactivates quickly as reaction pressure drops to 0 psig. Benzene is in vapor phase as the reaction pressure is 0 psig.

It was found that the feed ratios only slightly affect the conversion and selectivity as the ratio of benzene to 1-deodecene is greater than 5.

Herein, reaction kinetics was studied by examining operating variables of WHSV and reaction temperature. A linear correlation between ln(1-X) and residence time (1/WHSV) was obtained, indicating that the reaction follows a first order rate expression. A plot of ln($k_1$) versus 1/T in the reaction temperature range of 393K-413K, wherein $k_1$ is the first order rate constant and T is the reaction temperature, yields an activation energy about 17.0 kcal/mol.

Example 4: Mordenite catalyst modified by a chemical vapor deposition of Si

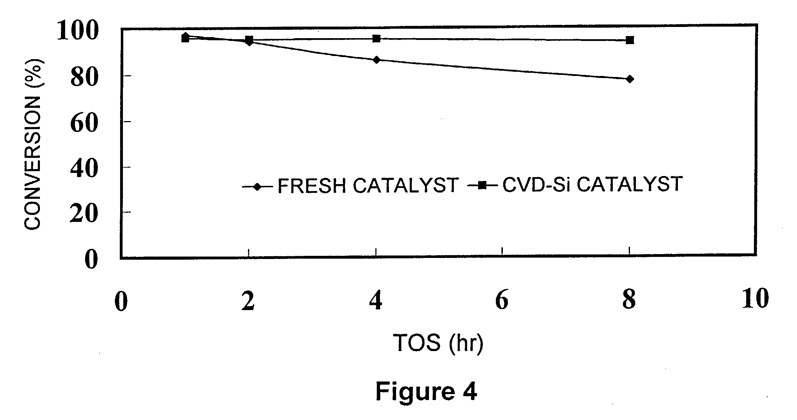

1.5 grams of 1/8 inch extrudate, 12-20 mesh catalyst was calcined in air at 813 K for 6 hr with a heating rate of about 120°C/hr and an air flow rate greater than 30 cc/min per gram of catalyst. The reactor was then cooled in a flowing $N_2$ atmosphere down to 140°C. Next, a liquid feed containing 99 parts by weight of benzene, 1 parts by weight of methanol and 0.02 parts by weight of $Si(OC_2H_5)_4$ was metered and introduced to the reactor at a flow rate of WHSV=5 hr$^{-1}$ together with a nitrogen flow at molar ratio of nitrogen to benzene of 10:1 for a period of one hour. The pressure in the reactor was 0 psig. The liquid feed was then stopped while maintaining the nitrogen flow for another one hour. The nitrogen flow was switched to air flow and the temperature was increased to 813 K for another four hours. The reactor was then cooled in a flowing $N_2$ atmosphere down to the desired reaction temperature for undergoing the alkylation. The alkylation conditions were 50 psig, 363 K, WHSV=16 hr$^{-1}$, $H_2$/HC=0.7. The feed composition was benzene:1-dodecene:1-decane=9:1:1. The results are shown in Fig. 4 together with the results from the run using a parent catalyst. As it can be seen from Fig. 4, the chemical vapor deposition of Si also improves the catalyst stability.

Although the present invention has been described with reference to specific details of certain embodiments thereof, it is not intended that such details should be regarded as limitations upon the scope of the invention except as and to the extent that they are included in the accompanying claims. Many modifications and variations are possible in light of the above disclosure.

## Claims

1.   A process for preparing a linear alkylbenzene by alkylating benzene with a C8-C18 long chain alpha olefin comprising flowing a hydrogen gas and a liquid feed comprising benzene and said olefin through a bed of a mordenite catalyst at a temperature ranging from 373 K to 523 K and at a pressure ranging from 0.5MPa to 5Mpa, and with a feed weight hourly space rate (WHSV) ranging from 2 to 20 hr$^{-1}$, wherein a molar ratio of benzene to said olefin in said liquid feed ranges between 2:1 and 20:1, and a molar ratio of said hydrogen to the liquid feed ranges between 0.1 and 10, **characterized in that** said mordenite catalyst is modified by being subjected to a steam pretreatment or a chemical vapor deposition of silicon prior to the alkylation.

2.   The process of claim 1, wherein said mordenite catalyst is modified by being subjected to the steam pretreatment, which comprises flowing a steam-containing gaseous mixture through a bed of mordenite catalyst at a temperature ranging from 500 K to 900 K, for a period from 0.5 to 10 hours, and wherein the steam-containing gaseous mixture has a steam partial pressure ranging from 30 to 200 mmHg, and the balance inert gas at room temperature.

3.   The process of claim 2, wherein said steam pretreatment comprises flowing said steam-containing gaseous mixture through said bed of mordenite catalyst at a flow rate of 30-1000 SCCC/min per gram of catalyst.

4.   The process of claim 3, wherein said steam pretreatment comprises flowing said steam-containing gaseous mixture through said bed of mordenite catalyst at a temperature of about 813 K for a period of about two hours, and at a flow rate of 30-200 SCCC/min per gram of catalyst, wherein the steam-containing gaseous mixture has a steam partial pressure of about 80 mmHg, and the balance air at room temperature.

5.   The process of claim 1, wherein said mordenite catalyst is modified by being subjected to the chemical vapor deposition of silicon, which comprises flowing a Si precursor-containing liquid and a carrier gas through a bed of mordenite catalyst at a temperature ranging from 353 K to 473 K, for a period from 0.5 to 6 hours, and wherein the Si precursor-containing liquid comprises an organic solvent or an organic solvent mixture and 0.01 to 1.0 parts of $Si(OC_2H_5)_4$ or $Si(OCH_3)_4$ based on the weight of the organic solvent or the organic solvent mixture.

6.   The process of claim 5, wherein the Si precursor-containing liquid comprises 0.02 to 0.2 parts of $Si(OC_2H_5)_4$ based on the weight of the organic solvent mixture comprising 80-99 wt% of benzene and 1-20 wt% of methanol.

7.   The process of claim 6, wherein the chemical vapor deposition of silicon comprises flowing the Si precursor-containing liquid and a carrier gas through a bed of mordenite catalyst at a temperature ranging from 393 K to 413 K, for a period from 1.0 to 5 hours.

8.   The process of claim 2, wherein said mordenite catalyst has a Si/Al ratio of 50-200 prior to said steam pretreatment.

9.   The process of claim 5, wherein said mordenite catalyst has a Si/Al ratio of 50-200 prior to said chemical vapor deposition of silicon.

10.   The process of claims 8 or 9, wherein said mordenite catalyst has a Si/Al ratio of about 90.

**11.** The process of claims 2 or 5, wherein said mordenite catalyst is in the particulate form having a particle size ranging from 4 to 30 mesh number.

**12.** The process of claim 11, wherein said mordenite catalyst has particle size ranging from 8 to 20 mesh number.

**13.** The process of claims 2 or 5, wherein said mordenite catalyst comprises about 30% by weight of $Al_2O_3$ binder.

**14.** The process of claim 1, wherein said C8-C18 long chain alpha olefin is 1-deodecene.

**Figure 1 (Prior Art)**

Figure 2

Figure 3

**Figure 4**

EP 1 249 440 A1

**European Patent Office**

## EUROPEAN SEARCH REPORT

Application Number

EP 01 10 8956

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 504 541 A (MONSANTO EUROPE SA) 23 September 1992 (1992-09-23) * column 4 - column 14 * | 1-4,8, 10-14 | C07C15/107 C07C2/66 B01J29/18 |

TECHNICAL FIELDS SEARCHED (Int.Cl.7)

C07C
B01J

The present search report has been drawn up for all claims

| Place of search | Date of completion of the search | Examiner |
|---|---|---|
| THE HAGUE | 21 August 2001 | Van Geyt, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

## ANNEX TO THE EUROPEAN SEARCH REPORT
## ON EUROPEAN PATENT APPLICATION NO.

EP 01 10 8956

This annex lists the patent family members relating to the patent documents cited in the above-mentioned European search report.
The members are as contained in the European Patent Office EDP file on
The European Patent Office is in no way liable for these particulars which are merely given for the purpose of information.

21-08-2001

| Patent document cited in search report | Publication date | Patent family member(s) | | Publication date |
|---|---|---|---|---|
| EP 0504541 A | 23-09-1992 | AT 168671 T | | 15-08-1998 |
| | | AU 657306 B | | 09-03-1995 |
| | | AU 1212592 A | | 24-09-1992 |
| | | BR 9200987 A | | 24-11-1992 |
| | | CA 2062560 A | | 22-09-1992 |
| | | CS 9200818 A | | 14-10-1992 |
| | | DE 69129843 D | | 27-08-1998 |
| | | DE 69129843 T | | 11-03-1999 |
| | | ES 2121776 T | | 16-12-1998 |
| | | HU 60704 A,B | | 28-10-1992 |
| | | JP 5117168 A | | 14-05-1993 |
| | | MX 9201263 A | | 01-10-1992 |
| | | US 5233111 A | | 03-08-1993 |

EPO FORM P0459

For more details about this annex : see Official Journal of the European Patent Office, No. 12/82